# DEMANDE DE BREVET EUROPEEN

(11) **EP 4 762 940 A1**
(43) Date de publication de la demande: **24.06.2026**
(21) Numéro de dépôt: 25224878.6
(22) Date de dépôt: 18.12.2025
(51) Int. Cl.: A23K 10/20, A23J 1/02, A23K 20/147, A01K 67/34

(54) **PROCÉDÉ DE VALORISATION DES LARVES D'INSECTES, COMPOSITION OBTENUE ET UTILISATION EN ALIMENTATION ANIMALE**

(30) Priorité: 19.12.2024 FR 2414755
(71) Demandeur: Mutatec, 84300 Cavaillon (FR)
(72) Inventeur: COSTIL, Jérôme, 13160 CHATEAURENARD (FR)
(74) Mandataire: Oak & Fox

(57) **Abrégé**

Procédé de préparation d'une composition de larves d'insectes à partir d'un milieu contenant des larves d'insectes vivantes et des particules de résidus de substrat comprenant au moins une étape de séparation aéraulique de manière à obtenir lesdites larves d'insectes d'une part et les particules de résidus de substrat présentant une densité différente de la densité des larves d'insectes d'autre part et une étape de traitement thermique desdites larves d'insectes à une température allant de 85°C à 105°C pendant une durée allant de 1 à 15 minutes et pâte d'insectes obtenue par ce procédé.

## Description

### Domaine technique de l'invention

La présente invention se rapporte au domaine de la valorisation des larves d'insectes. Plus particulièrement, elle concerne un procédé de préparation d'une composition à partir de larves d'insectes, la composition humide obtenue ainsi que son utilisation en tant que produit pour l'alimentation animale, et notamment l'aquaculture et l'alimentation des animaux de compagnie.

### État de la technique

Avec l'augmentation de la population mondiale et la tension sur les ressources naturelles qui en résulte, le recyclage des résidus agricoles et des industries agro-alimentaires ainsi que la lutte contre le gaspillage alimentaire sont plus que jamais à l'ordre du jour. Après des décennies durant lesquelles l'élimination de ces résidus (produits hors calibre, invendus ou périmés, résidus des industries de première transformation) a essentiellement consisté à s'en débarrasser dans des décharges, les acteurs des filières agricoles se sont progressivement intéressés à leur valorisation. En particulier, des solutions de valorisation par compostage ou par méthani-sation se sont développées. Depuis une dizaine d'années, les recherches se sont dirigées vers un mode de valorisation plus noble, permettant de recycler les éléments nutritifs, et particulièrement les protéines, pour l'alimentation des animaux : la bioconversion de ces résidus au moyen de larves d'insectes.

En effet, l'accroissement de la population mondiale et de son niveau de vie pèsent aussi sur la consommation globale de viande et de poisson. Or, l'un des facteurs limitant la production d'animaux d'élevage est la disponibilité des protéines à incorporer à leur ration, qui proviennent pour l'essentiel de la culture du soja ou de farines de poissons issus de la pêche océanique et dont le caractère durable est de plus en plus remis en question.

L'élevage d'insectes à la fois pour recycler les résidus agricoles et agro-alimentaires et comme source de protéines pour l'alimentation animale est une solution recommandée par l'Organisation des Nations Unies pour l'alimentation et l'agriculture (FAO). L'utilisation en particulier des larves de l'espèce Hermetia illucens, s'est révélée être une solution technique intéressante.

L'évolution de la réglementation européenne de 2017 (Règlement UE 2017/893 suivi par d'autres) qui a autorisé l'introduction de protéines d'insectes dans les formulations pour l'alimentation des animaux a ouvert la possibilité de développer sur ces bases techniques une nouvelle économie industrielle circulaire.

Ainsi, la demande de brevet WO2022177422A1 décrit déjà un procédé de préparation d'une pâte d'insectes. Ce procédé est mis en œuvre à partir de larves lavées et comprend une étape de chauffage de la pâte suivie d'un refroidissement en deux étapes, il ne décrit pas le procédé selon l'invention comprenant une étape de séparation aéraulique suivie d'un traitement thermique.

Il demeure néanmoins toujours un besoin de nouveaux procédés de préparation de compositions pour l'alimentation animale à base de larves d'insectes notamment utilisables sous forme « humide » dans la fabrication de formulations pour animaux, et tout particulièrement des animaux de compagnie.

Concernant l'aspect microbiologique, les fabricants de produits finis humides ne requièrent généralement pas de spécifications particulières relatives à la matière première protéinée car les lignes de production du produit final intègrent de manière systématique une étape de stérilisation.

De manière plus générale la catégorie 3 des sous-produits animaux (selon le règlement européen (CE) n°1069/2009) à laquelle appartiennent les compositions humides à base de larves d'insectes n'est pas sujette à des contraintes réglementaires particulières sur le plan microbiologique.

Néanmoins il est toujours recherché des compositions humides à base de larves d'insectes présentant un « bon état sanitaire » et une innocuité comparable à celle des abats ou viandes séparées mécaniquement disponibles sur le marché.

### Présentation de l'invention

La solution proposée par la présente invention est un procédé de préparation d'une composition de larves d'insectes pour la fabrication de produits pour l'alimentation des animaux à partir d'un milieu contenant des larves d'insectes vivantes et des particules de résidus de substrat, ledit procédé comprenant au moins les étapes suivantes, dans cet ordre :
- une étape de séparation aéraulique de manière à obtenir lesdites larves d'insectes d'une part et les particules de résidus de substrat présentant une densité différente de la densité des larves d'insectes d'autre part ;
- une étape de récupération des larves d'insectes ;
- une étape de traitement thermique desdites larves d'insectes à une température allant de 85°C à 105°C, de préférence pendant une durée allant de 1 à 15 minutes, de préférence allant de 4 à 12 minutes, l'étape de traitement thermique étant effectuée au moyen de vapeur sèche, ladite vapeur sèche étant de la vapeur injectée sous pression dans une enceinte, les larves d'insectes étant entrainées en déplacement dans ladite enceinte entre une entrée d'enceinte et une sortie d'enceinte.

De manière surprenante et avantageuse, le procédé selon l'invention permet l'obtention d'une composition présentant une innocuité de l'ordre de celle des abats et viandes séparées mécaniquement disponibles sur le marché. Or, à la différence de ces derniers, la composition obtenue selon l'invention est fabriquée à partir des larves entières c'est-à-dire incluant à la fois les cuticules externes et le tube digestif des larves avec leur contenu, ce qui confère à ladite composition une charge microbienne naturelle plus élevée que les viandes et abats classiques. Or cette charge microbienne pourrait être de nature à présenter un risque sur la stabilité de leur conservation.

De manière préférée, ladite pression d'injection de la vapeur est comprise de préférence entre 1 et 4 bars.

Le procédé selon la présente invention permet d'éviter un lavage des larves d'insectes à l'eau, ce qui permet à la fois d'économiser de l'eau et d'éviter la mise en place d'étapes de séchage ultérieures, ainsi que le traitement des eaux usées.

Plus généralement le procédé selon l'invention est relativement économe en termes d'énergie mise en œuvre et permet de fabriquer une composition de bonne qualité en un temps raisonnable.

La composition obtenue à l'issue du traitement thermique peut être utilisée directement dans la fabrication de produits pour l'alimentation des animaux.

Avantageusement, l'étape de traitement thermique est suivie d'une étape de broyage énergique de manière à obtenir la composition de larves d'insectes sous forme d'une pâte. Cette variante, préférée, du procédé selon l'invention permet de proposer aux industriels un produit neutre où la présence de larves d'insectes n'est pas directement reconnaissable. Ainsi, les larves d'insectes peuvent être intégrées dans des aliments pour animaux prêts à la consommation sans que les aversions instinctives ou culturelles vis-à-vis des insectes ne provoquent leur rejet.

Selon un mode préféré, l'étape de traitement thermique du procédé de l'invention est suivie d'une étape de broyage de manière à obtenir une composition comprenant plus de 85% de particules de granulométrie inférieure à 3 10⁻⁴ m et moins de 0,5 % de particules de granulométrie supérieure à 2 10⁻³ m.

Selon un autre mode préféré, la composition obtenue est refroidie à une température de 4°C en une durée inférieure ou égale à 10 heures puis congelée à une température inférieure -18°C, de préférence allant de -18°C à -40°C.

Le procédé selon la présente invention, en particulier lorsqu'il comprend les deux modes préférés présentés ci-dessus, permet d'obtenir une composition humide, sous forme de pâte, qui ne varie pas d'une production à l'autre et qui est stable pendant plusieurs mois jusqu'à son utilisation. Ceci permet l'utilisation de cette pâte congelée de manière différée, plusieurs semaines voire mois après sa préparation.

L'invention vise aussi la composition susceptible d'être obtenue par le procédé selon l'invention, ladite composition étant une composition humide comprenant avantageusement de 65 à 75 % en poids d'eau par rapport au poids total de la composition, ladite composition étant éventuellement congelée.

L'invention vise encore l'utilisation de la composition selon l'invention pour l'alimentation animale, de préférence pour l'aquaculture ou l'alimentation des animaux de compagnie.

### Présentation d'une figure unique

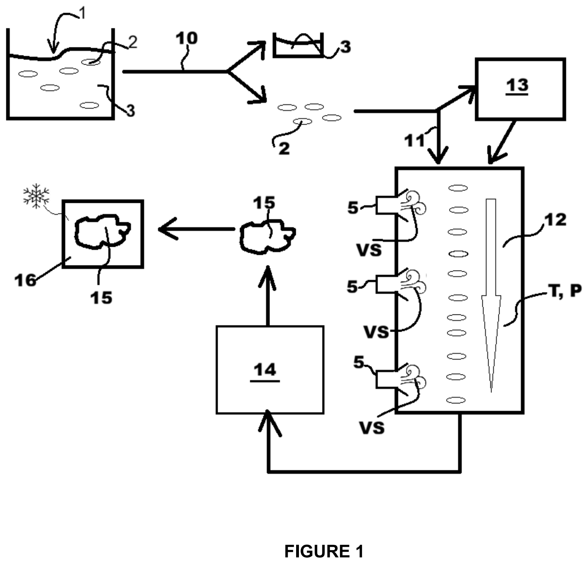

L'invention sera davantage comprise à la lumière de la description d'un mode de mise en œuvre préféré, illustré schématiquement par la figure 1 définie de la façon suivante :

La figure 1 illustre schématiquement les étapes d'un procédé conforme à l'invention permettant d'obtenir une composition pour l'alimentation animale, à partir de larves d'insectes.

### Description détaillée

Le procédé de préparation selon la présente invention est mis en oeuvre à partir d'un milieu 1 comprenant des larves d'insectes vivantes 2 et des particules de résidus de substrat 3.

Les larves d'insectes sont élevées dans un milieu dit « milieu d'élevage » ou « substrat de nourrissage » ; dans le présent texte, ces deux expressions sont utilisées pour désigner le même milieu. A la fin de la période d'élevage ce milieu comprend, outre les larves, des particules de résidus de substrats.

Les particules de résidus de substrat sont constituées de particules de de substrat de nourrissage digéré, c'est-à-dire les déjections des larves, encore appelées « frass », ainsi que de particules du substrat de nourrissage des larves non digestibles telles que des fibres cellulosiques ou les parties ligneuses.

Les larves d'insectes utilisées sont en règle générale âgées de moins de 15 jours. Les larves d'insectes sont introduites environ 5 à 7 jours après éclosion des œufs dans le substrat de nourrissage où elles sont élevées pendant 6 à 10 jours. Les larves utilisées dans le procédé de l'invention sont vivantes.

Toutes sortes de larves d'insectes peuvent être utilisées dans le procédé selon l'invention, de manière préférée les larves d'insectes sont celles de l'espèce *Hermetia illucens* (dite mouche soldat noire).

Généralement, les larves d'insectes de l'espèce *Hermetia illucens* présentent, au moment de la mise en œuvre du procédé de préparation selon l'invention, un poids compris entre 8 10⁻² g et 2,5 10⁻¹ g, une longueur comprise entre 10 10⁻³ m et 20 10⁻³ m et une largeur comprise entre 3 10⁻³ m et 6 10⁻³ m.

Comme déjà mentionné, le procédé selon l'invention comprend au moins les deux étapes suivantes : une étape de séparation aéraulique 10 et une étape de traitement thermique 20.

En fonction de la qualité du milieu contenant les larves d'insectes, qui dépend essentiellement de la composition initiale du milieu d'élevage et de l'avancement de sa digestion par les larves, l'étape de séparation aéraulique, peut être précédée d'au moins une étape de séparation préalable notamment par tamisage.

### Séparation aéraulique

La séparation aéraulique 10 vise à séparer les larves d'insectes des particules de résidus de substrat c'est-à-dire les différentes particules présentes dans le milieu en fonction de leur densité, au moyen d'un flux d'air.

A l'issue de l'étape de séparation aéraulique, les larves d'insectes sont substantiellement exemptes de particules de résidus de substrat, c'est-à-dire que le taux de particules de résidus de substrat est inférieur à 5% en poids sec de l'ensemble larves d'insectes et particules de résidus de substrat.

Bien entendu, les différents paramètres de fonctionnement du séparateur aéraulique à appliquer vont dépendre du modèle de séparateur aéraulique choisi. Il relève des compétences de l'homme du métier de définir les paramètres de fonctionnement du séparateur aéraulique en fonction de sa géométrie pour mettre en œuvre la séparation aéraulique définie dans la présente invention.

Généralement le paramètre de fonctionnement est essentiellement la vitesse de l'air, avantageusement la séparation aéraulique est effectuée au moyen d'un flux d'air dont la vitesse va de 5 à 8 m/s.

La vitesse de l'air (fonction du débit d'air et de la section de passage de l'appareil) est le principal paramètre physique de réglage de l'équipement qui détermine l'efficacité de la séparation aéraulique.

En outre, la séparation aéraulique est généralement effectuée sur des particules de résidus substrat présentant une teneur en eau allant de 40 à 60 % et des larves d'insectes présentant une teneur en eau allant de 65 à 75 %.

Lorsque les larves d'insectes sont des larves de mouches *Hermetia illucens,* alors l'étape de séparation aéraulique est effectuée de manière à séparer les larves d'insectes présentant une densité allant de 0,6 à 0.8 g/cm³ d'une part et les particules de résidus de substrat présentant une densité inférieure à 0,6 g/cm³ d'autre part.

Le traitement des larves d'insectes génère des odeurs qui peuvent être une source de gêne pour les opérateurs, aussi, selon un mode préféré de l'invention, la séparation aéraulique est effectuée dans un dispositif dans lequel l'air circule en circuit fermé.

Ce mode préféré présente l'avantage de réduire les odeurs générées par le traitement des larves d'insectes et de réduire les opérations postérieures liées au traitement de l'air.

A l'issue de cette étape, les larves d'insectes sont récupérées, cette étape peut consister en un simple convoyage , par exemple au moyen d'une trémie, de la zone où est effectuée l'étape de séparation aéraulique à la zone où est effectuée l'étape de traitement thermique.

### Traitement thermique

L'étape de séparation aéraulique est suivie d'une étape de traitement thermique. L'étape de séparation aéraulique et l'étape de traitement thermique peuvent être réalisées sans étape de pause intermédiaire ou en effectuant une étape de pause intermédiaire comprise entre 1 heure et 24 heures.

Avantageusement, l'étape de séparation aéraulique et l'étape de traitement thermique sont réalisées sans étape de pause intermédiaire.

L'étape de traitement thermique est réalisée à une température T allant de 85°C à 105°C, de préférence 95°C pendant une durée allant de 1 à 15 minutes de préférence allant de 4 à 12 minutes.

Cette température T correspond à la température des larves traitées.

Ce traitement, à cette température, permet à la fois l'abattage instantané des larves d'insectes, l'inactivation des enzymes digestives présentes dans le tube digestif des larves, et leur hygiénisation c'est-à-dire la diminution drastique de la charge microbienne des larves.

De préférence, ce traitement est effectué au moyen de vapeur sèche VS.

Par « vapeur sèche » ou « vapeur saturée » on entend de la vapeur d'eau chauffée à haute température, supérieure à 95°C et idéalement 135°C, et contenant moins de 5%, en volume d'eau à l'état liquide, injectée sous pression dans une enceinte.

Ainsi la vapeur sèche VS utilisée dans le traitement thermique selon l'invention est généralement injectée sous pression dans une enceinte à une température allant de 100°C à 135 °C.

L'étape de traitement thermique au moyen de vapeur sèche présente l'avantage d'éviter que les larves se chargent en eau, diminuant la teneur en matière sèche du produit, et permet en conséquence d'éviter la mise en oeuvre d'une étape d'égouttage ou de séchage. En outre l'injection de vapeur sèche sous pression P, généralement comprise entre 1 et 4 bars, permet de mettre les larves en suspension dans le courant de vapeur, ce qui garantit un échange de chaleur optimal, c'est-à-dire l'enveloppement direct de chaque larve par le fluide de chauffage, ce qui conduit d'abord à un abattage instantané des larves et ensuite à leur hygiénisation.

Selon un premier mode particulier, l'étape de traitement thermique est effectuée de la manière suivante. Les larves propres sont introduites dans une enceinte, par exemple un cylindre métallique fermé 4 ayant une pente de 10 % à 20 % *via* une trémie située à l'extrémité inférieure dudit cylindre. Le cylindre comprend une vis de diamètre à peine inférieur à celui du cylindre, qui tourne lentement pour brasser délicatement la masse de larves tout en les dirigeant vers l'extrémité supérieure du cylindre. Des buses 5 sont disposées à intervalles réguliers le long de la génératrice inférieure du cylindre pour injecter dans le cylindre (dans l'enceinte) de la vapeur sèche VS sous pression P sous la masse de larves, ce qui contribue à disperser les larves. Une ouverture située à l'extrémité supérieure du cylindre permet de récupérer les larves traitées.

Selon un deuxième mode particulier, l'étape de traitement thermique est réalisée au moyen d'une enceinte différente, comportant un cylindre rotatif horizontal muni d'une vis sans âme et de buses d'injection de vapeur situées sur la paroi dudit cylindre. Selon ce deuxième mode particulier, les larves sont introduites à une extrémité du cylindre, et le mouvement de rotation du cylindre provoque le brassage de la masse de larves dans le volume du cylindre. Les larves sont ainsi brassées et mises en contact avec le flux de vapeur tout en avançant vers l'extrémité opposée du cylindre, où elles sont récupérées.

Comme déjà mentionné, le procédé selon l'invention comprenant au moins les deux étapes suivantes : une étape de séparation aéraulique et un traitement thermique permet l'obtention d'une composition de larves d'insectes présentant un bon état sanitaire et une innocuité identique à celle des produits carnés disponibles sur le marché telles que les abats ou les viandes séparées mécaniquement.

### Etape(s) préalable(s) de tamisage

L'étape de séparation aéraulique, peut être précédée d'au moins une étape de séparation préalable notamment par tamisage 13.

En effet, la composition du milieu utilisé dans le procédé selon l'invention peut varier en fonction de la nature des résidus organiques initialement présents dans le milieu d'élevage des larves d'insectes. En conséquence, en fonction de la proportion et de la taille des particules de résidus de substrat dans le milieu à la fin de la période d'élevage des larves, la séparation larves/particules de résidus de substrat peut être effectuée au moyen d'une seule étape de séparation aéraulique ou au moyen d'une étape de séparation aéraulique précédée d'au moins une étape de tamisage.

Avantageusement, l'étape de séparation aéraulique est précédée d'au moins une étape de séparation par tamisage 13 des larves d'insectes, de préférence au moyen d'un tamis, avantageusement vibrant, de mailles de largeur allant de 10⁻³ m à 10⁻² m et de longueur allant de 2,5 10⁻² m à 6 10⁻² m. L'étape de tamisage préalable permet ainsi de réaliser l'étape de séparation aéraulique dans des conditions plus favorables, sur un milieu déjà débarrassé d'une partie des particules de résidus de substrat, améliorant ainsi la performance de l'étape de séparation aéraulique. L'étape de tamisage préalable permet d'utiliser un séparateur aéraulique de plus petites dimensions et de moindre consommation d'énergie, tout en assurant un même résultat et/ou une meilleure propreté des larves.

Selon un mode préféré de l'invention, un tamisage en deux étapes est effectué avant l'étape de séparation aéraulique.

De manière particulière, le tamisage en deux étapes est effectué au moyen de deux tamis : un tamis à grosses mailles positionné à l'étage supérieur et un tamis à mailles plus fines positionné à l'étage inférieur.

Lors de cette mise en œuvre de ce procédé, le substrat qui comprend les larves d'insectes et les particules de résidus de substrat est versé à l'entrée du premier tamis longitudinal vibrant, situé à l'étage supérieur. La vibration du tamis est configurée pour permettre au milieu à traiter de se répandre sur la largeur du tamis, d'en séparer/disperser les constituants puis de les acheminer vers l'extrémité du tamis.

Le tamis positionné à l'étage supérieur est à mailles rectangulaires d'une taille légèrement supérieure à celle des larves, par exemple de largeur allant de 5 10⁻³ m à 10⁻² m et de longueur allant de 2,5 10⁻² m à 6 10⁻² m. Ainsi les particules de résidus de substrats plus grandes que ces dimensions, telles que les morceaux de résidus organiques non digérés, les amas de fibres, les noyaux ou les pelures de fruits, restent sur le tamis et sont évacuées en extrémité dudit tamis. Les larves et les fines particules de résidus de substrat telles que les enveloppes de céréales de petites dimensions passent progressivement à travers les mailles et tombent sur le deuxième tamis vibrant à mailles plus fines positionné à l'étage inférieur.

Le deuxième tamis vibrant est placé perpendiculairement au premier, de sorte que les particules qui passent à travers les mailles du premier tamis sont immédiatement réparties sur toute la largeur du deuxième tamis. Le deuxième tamis positionné à l'étage inférieur est à mailles rectangulaires d'une taille légèrement inférieure à celle des larves, par exemple de largeur allant de 1 10⁻³ m à 5 10⁻³ m et de longueur allant de 2,5 10⁻² m à 6 10⁻² m.

La fréquence de vibration et la longueur du tamis sont réglées de manière à ce que le mouvement provoque progressivement la séparation des larves et des particules de résidus de substrat ainsi que l'acheminement des larves et des particules de résidus de substrat de taille voisine de la taille des larves vers l'extrémité du tamis, tandis que les particules plus petites tombent à travers les mailles.

Les larves et les particules de résidus de substrat de taille voisine de la taille des larves sont récupérées en extrémité du tamis.

Avantageusement, lorsque les larves d'insectes sont de l'espèce Her*metia illucens,* l'étape de séparation aéraulique est précédée d'une séparation par tamisage des larves d'insectes comprenant au moins les étapes suivantes, dans cet ordre :

tamisage grossier au moyen d'un tamis de mailles rectangulaires de largeur allant de 5 10⁻³ m à 10⁻² m et de longueur allant de 2,5 10⁻² m à 6 10⁻² m,

tamisage fin au moyen d'un tamis de mailles rectangulaires de largeur allant de 10⁻³ m à 5 10⁻³ m et de longueur allant de 2,5 10⁻² m à 6. 10⁻² m.

### Broyage

Selon un mode préféré, une étape de broyage 14 est réalisée après l'étape de traitement thermique.

La composition utilisée pour l'étape de broyage comprend avantageusement de 65 à 75 % en poids d'eau par rapport au poids total de la composition.

Avantageusement, l'étape de traitement thermique 12 est suivie d'une étape de broyage 14 de manière à obtenir une composition comprenant plus de 85% de particules de granulométrie inférieure à 3 10⁻⁴ m et moins de 0,5 % de particules de granulométrie supérieure à 2 10⁻³ m.

L'étape de broyage 14 est généralement effectuée par une action rotative à haute vitesse, notamment par l'action de plusieurs étages de couteaux tournant à haute vitesse devant une succession de grilles de taille de mailles décroissante. Ainsi compte tenu du contenu en eau et en matière grasse des larves d'insectes, l'étape de broyage conduit à la formation d'une pâte 15 qui est une émulsion stable, c'est-à-dire qui ne déphase pas même après plus de 48 heures de stockage, et même après décongélation de la pâte congelée.

Lorsque l'étape de broyage 14 est réalisée immédiatement après celle du traitement thermique, sans interruption, la température de l'émulsion 15 va de 85 à 95°C.

La composition est ensuite refroidie ou laissée refroidir. Avantageusement, afin de préserver le bon état sanitaire et de fraîcheur de la composition, la durée du refroidissement jusqu'à atteindre 4°C doit être inférieur à 10 heures.

La composition obtenue est une composition humide comprenant avantageusement de 65 à 75 % en poids d'eau par rapport au poids total de la composition.

### Congélation

La pâte obtenue est de préférence conservée sous forme congelée à une température inférieure à -18°C et de préférence allant de -18°C à - 40°C. La congélation 16 permet d'éviter tout développement microbien et la reprise de toutes réactions enzymatiques et physico-chimiques.

Les exemples qui suivent visent à illustrer l'invention sans en limiter la portée.

### Exemples

### Exemple 1- Mise en œuvre du procédé selon l'invention

Des larves de mouche de l'espèce *Hermetia illucens* ont été introduites à l'âge de 5 jours après éclosion des œufs dans un substrat de nourrissage composés de divers résidus organiques d'origine végétale finement broyés. Après 8 jours d'engraissement, ces larves ont digéré la totalité du substrat et ont grossi jusqu'à atteindre un poids moyen de 10⁻¹ g.

6 10⁶ g (6000 kg) du milieu comprenant les larves et des particules de résidus de substrat (résidus de digestion du substrat) ont été prélevés.

Ces 6 10⁶ g de milieu ont été traités par le procédé conforme à l'invention en 6 étapes présenté ci-dessous.

Le milieu a été introduit dans un système de tamis vibrants à deux étages.

### Etape 1

Le système de tamis comprend, à l'étage supérieur, un tamis vibrant de forme rectangulaire à mailles rectangulaires de taille 8 10⁻³ m X 5 10⁻² m, permettant un déplacement des larves dans le sens longitudinal du tamis.

Ce premier tamisage permet de séparer et d'éliminer les particules de résidus de substrat non digéré tels que des croûtes, amas de fibres, morceaux de noyaux, qui sont retenus au-dessus du tamis tandis que les larves et les particules de résidus de substrat plus fines telles que les particules de substrat digéré traversent le tamis pour rejoindre l'étage inférieur.

### Etape 2

Le système de tamis comprend à l'étage inférieur un tamis vibrant de forme rectangulaire, placé perpendiculairement au tamis de l'étage supérieur et présentant des mailles rectangulaires de taille 1,5 10⁻³ m X 5,2 10⁻² m permettant un déplacement des larves dans le sens longitudinal du tamis.

Les particules de résidus de substrat digéré plus fines traversent ce second tamis, tandis que les larves et les particules de résidus de substrat de dimensions comparable aux larves elles-mêmes sont retenus au-dessus et sont dirigées vers une trémie de récupération. Les larves ainsi séparées restent souillées par des particules de résidus de substrat de dimensions comparable aux larves elles-mêmes.

A l'issue de ces deux tamisages, 2,9 10⁶ g de matières comprenant majoritairement des larves mais aussi des particules de résidus de substrat de dimensions équivalentes à celles de larves ont été récupérées.

### Etape 3

Ces 2,9 10⁶ g de matières ont été introduites dans un séparateur aéraulique, commercialisé par la SAS RITEC sous la référence séparateur ZZ 160-500 où le mélange de larves et de résidus est déversé dans un courant d'air ayant une vitesse de l'air de 5 à 8 m/s.

Ce séparateur aéraulique met en œuvre un flux d'air ascendant à l'intérieur d'un conduit vertical en zigzag. La configuration dudit conduit permet d'allonger le parcours des particules et d'accélérer la vitesse de l'air.

Selon cette étape, le milieu contenant des larves et des particules de résidus de substrat de dimensions comparable aux larves elles-mêmes est introduit à l'extrémité supérieure du conduit vertical et entre au contact du flux d'air ascendant.

Ainsi, les particules légères de densité inférieure à 6 10⁵ g/m³ sont entraînées par le flux d'air ascendant et récupérées dans un cyclone positionné à distance. Les particules légères sont récupérées au fond du cyclone où une vanne-écluse a été placée.

L'air débarrassé des particules de résidus de substrat, récupéré en haut du cyclone est renvoyé *via* un circuit fermé dans la partie inférieure du conduit en zig-zag.

Les larves ainsi nettoyées sont évacuées via une vanne-écluse positionnée en -dessous du conduit en zig-zag.

Cette séparation aéraulique a permis de séparer les larves de densité d'environ 6 à 8 10⁵ g/m³ (0,6 à 0,8 tonne /m³) des particules de densité inférieure et ainsi de récupérer 2,55 10⁶ g de larves.

### Etape 4

Les 2,55 10⁶ g de larves ont ensuite subi un traitement thermique à la vapeur sèche. La vapeur sèche à 135°C a été introduite à un débit tel que la température dans l'enceinte de l'appareil soit maintenue à 95°C en permanence. Le débit d'alimentation de l'appareil en larves et la vitesse de rotation de la vis interne qui provoque l'avancement de la masse de larves dans l'appareil ont été réglés de telle façon que le temps de séjour des larves dans l'enceinte de l'appareil qui était maintenue à 95 °C soit de 6 minutes.

### Etape 5

Les larves ont ensuite été broyées au moyen d'un appareil de type Affineur KS FD175 type 112 # 35620 KARL SCHNELL à une vitesse de 2970 trs/mn et avec un jeu de grilles de taille décroissante de 10⁻² m à 4 10⁻³ m. Du fait de la vitesse élevée de rotation de l'appareil et du contenu en matières grasses des larves, le produit obtenu se présente sous la forme d'une émulsion épaisse, c'est-à-dire une pâte, avec une granulométrie des particules pour 85% inférieure à 3 10⁻⁴ m et pour moins de 0,5 % supérieure à 2 10⁻³ m.

A l'issue de cette étape, il n'est pas possible de reconnaitre à l'œil nu la présence de morceaux de larves. La température de l'émulsion à la sortie de l'appareil est de 90-95°C.

### Etape 6

L'émulsion est ensuite conservée par congélation -18°C.

Afin de prévenir tout risque de dégradation du produit ou de développement microbien, le refroidissement de l'émulsion entre 90-95°C et 4°C, est effectué en moins de 10 heures.

### Exemple 2-Détermination de la charge microbienne

Les larves d'*Hermetia illucens* se nourrissent de matières organiques dégradées et sont de ce fait immergées dans une vaste communauté de micro-organismes. Les bactéries présentes sur les cuticules extérieures et dans le tube digestif des larves appartiennent à différents groupes taxonomiques, dont certains peuvent présenter un risque pour la sécurité alimentaire.

Le tableau 1 présente les données microbiologiques en UFC/g mesurées sur des échantillons prélevés à différentes étapes du procédé présenté à l'exemple 1.

L'échantillon A correspond à un échantillon de larves vivantes prélevé avant l'étape 3 (séparation aéraulique) de l'exemple 1.

L'échantillon B correspond à un échantillon prélevé à la fin de l'étape 4 (traitement thermique) de l'exemple 1.

**Tableau 1**

| | Moyenne dans les larves vivantes A (nombre d'échantillons) | Moyenne des larves traitées B |
|---|---|---|
| Entérobactéries à 37°C | 1358238 (42) | < 100 |
| Clostridium perfringens | 243 (41) | < 10 |
| Bacillus cereus présomptifs | 2 205 (42) | < 100 |
| E. coli ß glucuronidase+ | 444 195 (41) | < 100 |
| Staphylocoques à coagulase | 24 753 (38) | < 10 |
| Levures Moisissures | 100 988 (42) | < 10 |

Les résultats montrent que le procédé selon l'invention permet de réduire significativement les teneurs en micro-organismes présentant des enjeux sanitaires.

### Exemple 3-Détermination de la quantité d'amines biogènes des compositions congelées

Les analyses ont été effectuées sur des échantillons de différents lots prélevés après l'étape 6 (congélation) de l'exemple 1 et pour différentes durées de conservation à -18°C.

La « fraîcheur » des échantillons est conventionnellement évaluée par le dosage des amines biogènes.

le tableau 2 présente les taux en amines biogènes en mg/kg des échantillons de composition congelée.

**Tableau 2**

| Echantillons | C | D | E | F |
|---|---|---|---|---|
| Durée de stockage à -18°C ( jours) | 15 | 10 | 7 | 28 |
| Histamine | <5 | 7,25 | 2,02 | 14,5 |
| Cadavérine | 7 | 7,82 | 2,57 | 20,3 |
| Putrescine | 6 | 15,8 | 6,91 | 32,2 |
| Spermine | 12 | 11 | 14,6 | 15,1 |
| Tyramine | <5 | 2,13 | <1 | 2,19 |
| Tryptamine | <5 | <5 | <5 | <5 |
| 2 Phenylethylamine | <5 | <1 | <1 | <1 |
| Spermidine | 52 | 62,4 | 49,4 | 58,5 |

On observe que les concentrations en amines biogènes restent faibles et dans tous les cas en dessous des seuils d'acceptabilité pour celles présentant de tels seuils : Histamine <30 ppm, Cadavérine<100 ppm, Putrescine<100 ppm.

### Exemple 3-Aspect visuel

L'évolution de la couleur des compositions selon l'invention non congelées a été comparée à celle d'une composition témoin non congelée n'ayant pas subi l'étape de traitement thermique.

Il a été observé que les compositions selon l'invention présentent une couleur claire et stable pendant une durée d'au moins 6h. Par contre, la composition témoin non traitée thermiquement se teinte instantanément en brun clair puis s'assombrit jusqu'à devenir noire en moins de 15 minutes.

Le procédé selon l'invention permet bien d'éviter un brunissement des compositions obtenues, significatif de la neutralisation des enzymes responsable de l'autolyse du produit.

## Revendications

1. Procédé de préparation d'une composition de larves d'insectes pour la fabrication de produits pour l'alimentation des animaux à partir d'un milieu contenant des larves d'insectes vivantes et des particules de résidus de substrat, ledit procédé comprenant au moins les étapes suivantes, dans cet ordre :
- une étape de séparation aéraulique de manière à obtenir lesdites larves d'insectes d'une part et les particules de résidus de substrat présentant une densité différente de la densité des larves d'insectes d'autre part ;
- une étape de récupération des larves d'insectes ;
- une étape de traitement thermique desdites larves d'insectes à une température allant de 85°C à 105°C, pendant une durée allant de 1 à 15 minutes de préférence allant de 4 à 12 minutes,
l'étape de traitement thermique étant effectuée au moyen de vapeur sèche, ladite vapeur sèche étant de la vapeur injectée sous pression dans une enceinte, les larves d'insectes étant entrainées en déplacement dans ladite enceinte entre une entrée d'enceinte et une sortie d'enceinte.

2. Procédé selon la revendication 1, **caractérisé en ce que** ladite pression d'injection de la vapeur est comprise de préférence entre 1 et 4 bars.

3. Procédé selon la revendication 1 ou 2 dans lequel l'étape de séparation aéraulique est précédée d'au moins une étape de séparation par tamisage des larves d'insectes, de préférence au moyen d'un tamis, avantageusement vibrant, de mailles de largeur allant de 10⁻³ m à 10⁻² m et de longueur allant de 2,5 10⁻² m à 6 10⁻² m.

4. Procédé selon l'une quelconque des revendications 1 à 3 dans lequel les larves d'insectes sont celles de l'espèce *Hermetia illucens.*

5. Procédé selon la revendication 4 dans lequel l'étape de séparation aéraulique est effectuée de manière à séparer les larves d'insectes présentant une densité allant de 0,6 à 0.8 g/cm³ d'une part et les particules de résidus de substrat présentant une densité inférieure à 0,6 g/cm³ d'autre part.

6. Procédé selon la revendication 4 ou 5 dans lequel l'étape de séparation aéraulique est précédée d'une séparation par tamisage des larves d'insectes comprenant au moins les étapes suivantes, dans cet ordre :
- tamisage grossier au moyen d'un tamis de mailles rectangulaires de largeur allant de 5 10⁻³ m à 10⁻² m et de longueur allant de 2,5 10⁻² m à 6 10⁻²m,
- tamisage fin au moyen d'un tamis de mailles rectangulaires de largeur allant de 10⁻³ m à 5 10⁻³ m et de longueur allant de 2,5 10⁻² m à 6. 10⁻² m.

7. Procédé selon l'une quelconque des revendications précédentes dans lequel l'étape de traitement thermique est suivie d'une étape de broyage de manière à obtenir une composition comprenant plus de 85% de particules de granulométrie inférieure à 3 10⁻⁴ m et moins de 0,5 % de particules de granulométrie supérieure à 2 10⁻³ m.

8. Procédé selon la revendication 1 dans lequel la composition obtenue est refroidie à une température de 4°C en une durée inférieure ou égale à 10 heures puis congelée à une température inférieure -18°C, de préférence allant de -18°C à -40°C.

9. Composition susceptible d'être obtenue par le procédé selon l'une quelconque des revendications 1 à 8, ladite composition étant une composition humide comprenant avantageusement de 65 à 75 % en poids d'eau par rapport au poids total de la composition, ladite composition étant éventuellement congelée.

10. Utilisation de la composition selon la revendication 9 pour l'alimentation animale, de préférence pour l'aquaculture ou l'alimentation des animaux de compagnie.
